# EUROPEAN PATENT SPECIFICATION

(11) **EP 4 436 527 B1**
(45) Date of publication and mention of the grant of the patent: **23.09.2026**
(21) Application number: 22839931.7
(22) Date of filing: 21.11.2022
(51) Int. Cl.: A61F 2/66

(54) **PROSTHETIC DEVICE HAVING A SACRIFICIAL ANODE**
PROTHETISCHE VORRICHTUNG MIT EINER OPFERANODE
DISPOSITIF PROTHÉTIQUE COMPRENANT UNE ANODE SACRIFICIELLE

(30) Priority: 26.11.2021 US 202163283355 P
(43) Date of publication of application: 02.10.2024
(73) Proprietor: OSSUR ICELAND EHF, 110 Reykjavik (IS)
(72) Inventor: SVAVARSSON, Ingi, 110 Reykjavik (IS)
(74) Representative: Winger
(86) International application number: PCT/US2022/050562
(87) International publication number: WO 2023/096857

(56) References cited:
- EP-B1- 0 048 247
- US-A- 5 383 935
- US-A1- 2005 192 636

## Description

### FIELD OF THE DISCLOSURE

The disclosure relates to a prosthetic device having a metal component with a sacrificial anode.

### BACKGROUND

Prosthetic devices having metal components may be exposed to harsh elements, such as rain, snow, sleet, and salt or chlorinated water. While many components in prosthetic devices are fabricated from composite and polymeric materials, some components are adapted to being formed by metals such as steel, aluminum, or titanium, or combinations of metal such as aluminum and steel, to indicate a few. The metal components provide superior properties over other materials, particularly in structural components involving repeated use. The metal components include adapters for connecting various components such as a prosthetic socket to a knee in a lower limb prosthetic system, clamps for securing to a pylon, adapter for a foot, valve assemblies for vacuum systems, and other known components used in a prosthetic system including a metal component. Document US5383935 (A) discloses a bone implant which includes materials that function as a galvanic couple. These include an anode, a porous wall and a biocompatible electrolyte. Invivo precipitation of calcium phosphate is induced to promote bone growth into the porous wall of the implant.

While these metal components may be weatherproof, the weatherproof device allows use in a wet and/or humid environment, such weatherproof devices do not allow submersion. Fresh water splashing against the metal component from any direction likely will have no harmful effect, yet such water excludes salt and chlorinated water.

The metal components should be waterproof. Waterproof connotes that there is permanent protection against harmful ingress of water. For example, in a prosthetic foot, even after submersion in water, a waterproof prosthetic foot's structural parts maintain their integrity and continue to provide full support, function, and durability. Even if waterproof, consistent exposure to hard elements may still cause corrosion of the components, leading to a deterioration in the appearance and structure of such components.

If a metal component is repeatedly exposed to harsh elements, its appearance and functionality may deteriorate. Corrosion of the metal components may occur, which may impede the performance of a prosthetic system and lead to a lack of trust by the user due to an unsightly appearance. For example, there may be an appearance of flattened, mushroomed or otherwise deformed machining marks due to corrosion, which may lead to more drastic peaks and valleys along with the metal component. In addition, high spots may arise that can generate high stresses leading to deformation of the peaks.

The corrosion may lead to material removal from one surface and generate debris that adheres to another surface. The corrosion can be mechanical and generated due to micromotion between two surfaces. It can also occur due to chemical reduction and oxidation processes that corrosively transfer material from one surface. It can also be a combination of these processes. The surface of the metal component may discolor. Changes in the color of the surfaces can occur due to corrosion, and there may be visible debris generated by such corrosion.

As it is desired to create minimal impact to the prosthetic device to assure that it is both weatherproof and waterproof, there is a need for means that do not significantly alter the structure and design of a prosthetic component to make it weatherproof and waterproof. It is desirable to configure existing prosthetic devices at minimal cost to assure they are resistant to harsh elements, including water, particularly salt and chlorinated water.

### SUMMARY

The embodiments described herein offer a solution to the problem of weatherproofing and waterproofing existing prosthetic devices having metal components.

According to a first embodiment, the prosthetic device includes a metal component having a sacrificial/galvanic anode. The sacrificing/galvanic anode, such as a zinc insert or pad, sacrifices itself to protect the steel (or other metal material) when in touch with the elements (water & especially saltwater). The anode corrodes more easily than the other parts in the prosthetic system. The corrosion products from the zinc are deposited on the steel, resealing it from the atmosphere and therefore stopping corrosion.

In this embodiment, the anode is preferably formed from zinc. The anode is pressed into an opening formed by the metal component and essentially can be placed anywhere on the surface where corrosion protection is needed. The embodiment is not limited to a single anode, and multiple anodes may be employed.

The provision of a sacrificial/galvanic anode with a metal component in a prosthetic system benefits in an improvement in appearance and structural integrity as there is no debris generated and material deterioration, and the possibility of surface stresses created by corrosion is mitigated.

The metal component is arranged to accommodate an anode without structurally modifying the metal component aside from providing means for placement of the anode. In a preferred embodiment, the metal component has an opening in which the anode is press-fitted into the opening. Preferably the anode is flush with an outer surface of the metal component so as not to protrude therefrom, yet providing sufficient exposure to harsh elements, and therefore protect the remainder of the metal component.

These and other features, aspects, and advantages of the embodiments of the disclosure will become readily apparent and better understood given the following description, appended claims, and accompanying drawings.

### BRIEF DESCRIPTION OF THE DRAWINGS

Fig. 1 is a perspective view of a prosthetic system, including a sacrificial anode connected to a metal component.
Fig. 2 is a perspective view of the metal component in Fig. 1.
Fig. 3 is a perspective view of the sacrificial anode in Fig. 1.
Fig. 4 is a partial perspective view of another embodiment of a prosthetic system, including a sacrificial anode connected to a metal component.
Fig. 5 is a perspective view of another embodiment of a prosthetic system, including at least two sacrificial anodes connected to metal components.
Fig. 6 is a perspective view of metal components secured to one another and having sacrificial anodes.

### DETAILED DESCRIPTION OF VARIOUS EMBODIMENTS

A better understanding of different embodiments of the disclosure may be had from the following description read with the accompanying drawings in which like reference characters refer to like elements.

While the disclosure is susceptible to various modifications and alternative constructions, certain illustrative embodiments are shown in the drawings and will be described below. It should be understood, however, there is no intention to limit the disclosure to the embodiments disclosed, but on the contrary, the intention is to cover all modifications, alternative constructions, combinations, and equivalents falling within the scope of the disclosure and defined by the appended claims.

It will be understood that, unless a term is defined in this patent to possess a described meaning, there is no intent to limit the meaning of such term, either expressly or indirectly, beyond its plain or ordinary meaning.

The term "comprises (or comprising)" means its definition as is standard in patent terminology, being an open-ended term that is generally synonymous with "includes," "having," or "containing." Although "comprises," "includes," "having," and "containing" and variations thereof are commonly-used open-ended terms.

In practicing the prosthetic device having a metal component with a sacrificial anode, such as a zinc insert or pad, the anode sacrifices itself to protect the steel or other metal material when in touch with harsh elements, such as salt or chlorinated water. The anode corrodes more easily than the other parts in the prosthetic system. The corrosion products from the zinc are deposited on the steel, resealing it from the atmosphere and stopping the corrosion of the metal components.

A sacrificial anode is used to prevent corrosion through cathodic protection. For example, zinc may be classified as a galvanic anode. The galvanic anode does not require an external power supply and instead relies on its natural potential of being more electro-negative than the potential of the structure being protected. For this disclosure, zinc will be considered a preferred material for the anode but can be replaced by other materials such as aluminum or magnesium or other suitable materials having protective properties.

The protective properties of zinc anode result from a strongly negative reduction potential, which is more negative than the metal it is protecting. Oxidants, which corrode metals, will oxidize the zinc anode rather than the protected metal structure, thus preventing corroding. Sacrificial anodes release electrons, slowly corroding the anode in place of the actual metal in use, which is less negatively charged.

When the zinc anode is placed close to another metal with a greater reduction potential, it makes the other metal the cathode of the electrochemical system. When an oxidant attacks the metal surface, electrons flow through the system and are removed from the zinc anode. An important metric of a zinc anode is its utilization factor, which specifies how much of the anode can be used before it loses its protective properties. The mass of a zinc anode needed for a particular protective application can be calculated.

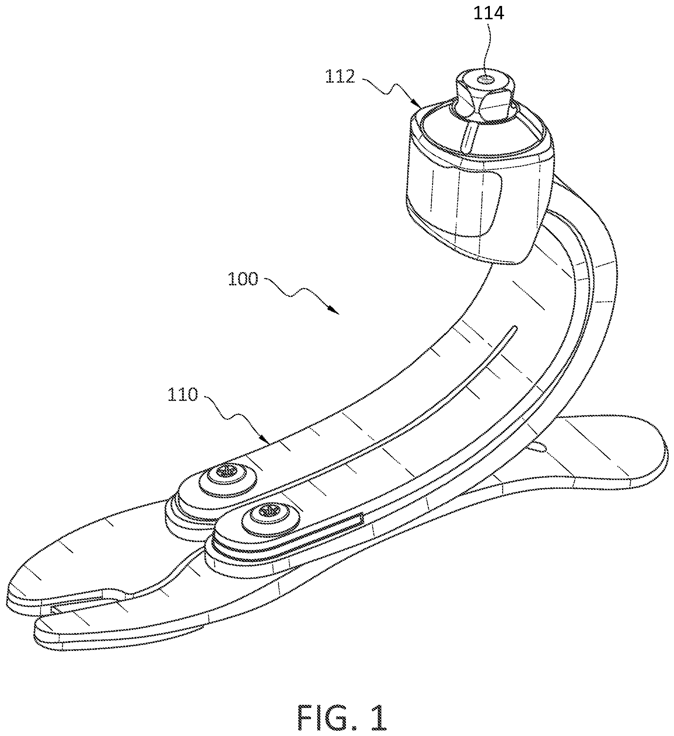

Fig. 1 illustrates a prosthetic system 100, including a prosthetic foot 110 with an adapter 112 secured or mounted to the prosthetic foot 110. The adapter 112 is preferably a pyramid adapter, a universal modular component used to connect to a variety of structural components. Typically, an adapter is a metal component made from titanium, aluminum, stainless steel, or any combination of such materials. The adapter 112 serves as an exemplary metal component for use with a sacrificial anode 114, which may be received an opening defined by the metal component or otherwise attached.

An example of an adapted in a prosthetic foot is found in U.S. patent 9,579,220, granted February 28, 2017, and a valve or valve components that may be formed from metal are found by example in U.S. patent 9,486,335, granted November 8, 2016.

The prosthetic foot is merely exemplary and the concepts described herein in connection to a sacrificial anode may be extended to prosthetic knees, sockets, and any feature having a metal component. Likewise, these concepts can be extended to other articles such as orthopedic devices and other wearable devices.

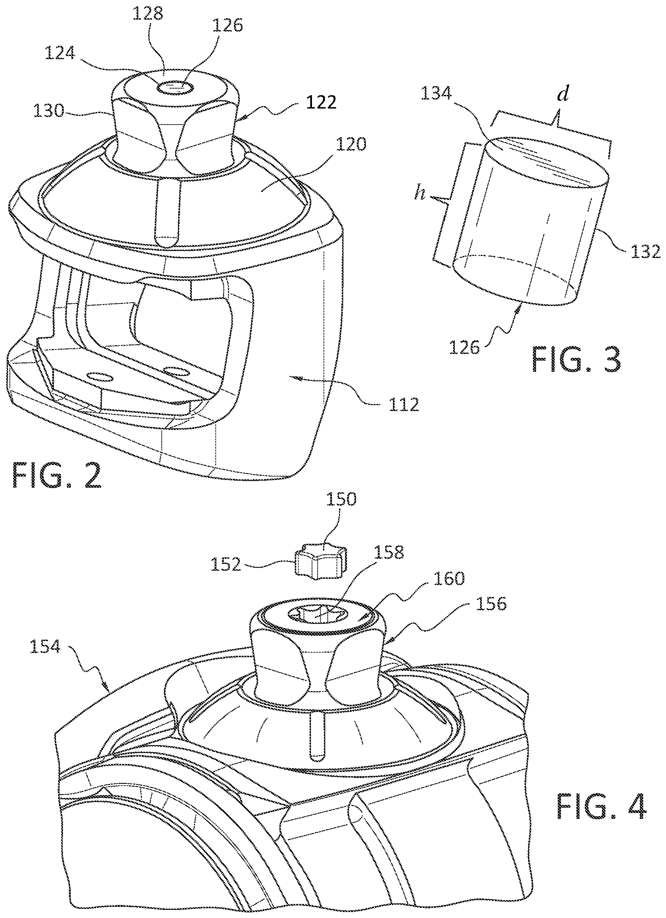

As further shown in Fig. 2, the anode 114 is substantially smaller than the metal adapter 112. In the exemplary embodiment, the adapter 112 includes an adapter top 120 having a locking part 122. The adapter top 120 defines an extension 130 at which the locking part 122 is located. The locking part 122 defines an opening 124 configured and dimensioned to receive the anode 126.

The opening is accorded its ordinary meaning of an aperture or gap, especially one allowing access or an open or empty space in or between things. The opening 124 defines or marks out the boundaries of a depth corresponding to a height h of the anode 126. The first end 132 of the anode 126 is preferably flush or at level with a surface 128 of the locking part 122, such that a surface area 134 of the first end 132 of the anode 126 is exposed. According to variations, the opening or the anode may be sized and configured to be recessed relative to the surface 128 or protruding outwardly from the surface. Either of such variations may be selected depending according to how the anode serves.

Referring to Figs. 2 and 3, the surface area 134 of the anode 126 as being substantially smaller than the surface area of the surface 128. For example, the surface area 134 of the anode is at least 50% less, or more preferably less than 25%, or even less than 10% than the surface area of the surface 128. While surface area 134 is useful for the extent of exposure to the anode 126, the anode 126 may also be selected due to its weight and capability to enhance its utilization factor.

The anode 126 defines a diameter d at least at the first end 132 that corresponds to a diameter or width of the opening 124 to which it opens along the surface 128. The first end 132 of the anode may define a symmetrical, uniform shape, and may be adapted to be replaced upon exhaustion, and a new anode can take its place.

The anode 126 is preferably secured to the adapter 112 in a tight-fitting relationship. For example, the anode may be press-fit, also known as an interference fit because it is fastened to the metal component by force or friction. The anode, as in the cylindrical form of Fig. 3, but also for other shapes selected for insertion, may be tightly fitted in the opening with the interference holding both parts in place.

The anode is preferably constructed from a zinc alloy, as zinc is known as a preferred material as an anode for corrosion resistance to saltwater. Preferably, the zinc alloy is at least 99% pure zinc. Alloys of aluminum or magnesium may be used as an anode.

Referring to Fig. 4, the anode 150 may define a shape 152 configured and dimensioned to tightly fit, as in press-fitting, into the engaging surface 160 within an enclosure 158 formed by a metal component 156 of prosthetic component 154. The enclosure or opening 158 may have a predetermined shape, not just cylindrical as in the embodiment of Figs. 2 and 3, may be likewise configured and dimensioned to have locking surfaces corresponding to the shape 152 of the anode 150.

For example, as illustrated, the shape 152 may have an undulating profile so that the anode 150 tightly fits or locks within the enclosure 158 due to corresponding shapes. Thus, the anode takes the shape of a star-like appearance but can take other known shapes to correspond to the shape of the enclosure defined by the metal component. The anode may be shaped to have threads or other locking surfaces that enable a mechanical fitting of the anode to a metal component, such as by screwing or a frictional fit with cooperating surfaces of the metal component as internal threads to the opening.

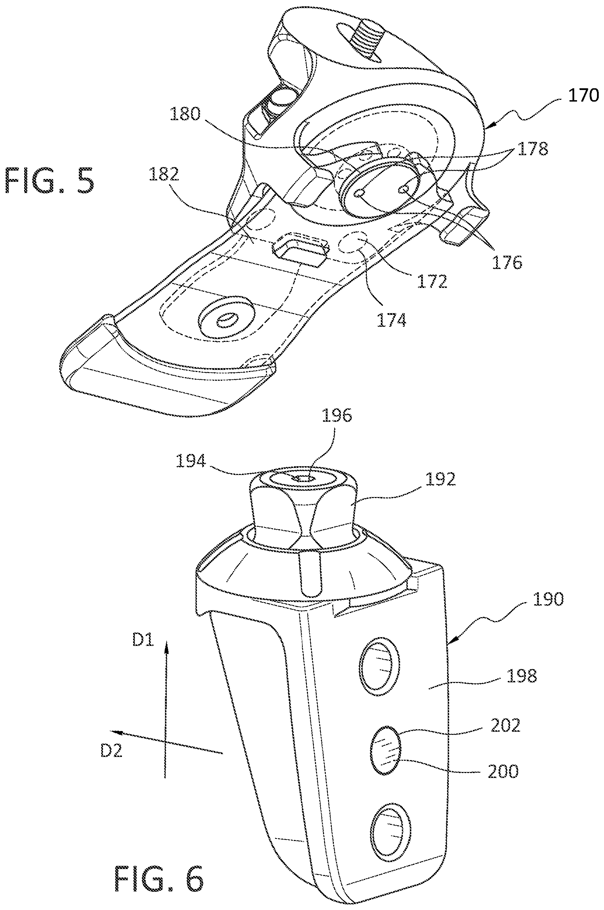

Referring to Fig. 5, a first metal component 180 may define first and second openings 178 configured and dimensioned to receive first and second anodes 176. In this embodiment, the prosthetic device 170 is a prosthetic foot, and the first metal component 180 is located on the sole or underside of the prosthetic foot, frequently exposed to harsh elements.

Fig. 5 illustrates how not only can a metal component have more than one or a plurality of anodes, but a prosthetic device can have more than one metal component with one or more sacrificial anodes. For example, a second metal component 182 defines at least one opening 174 configured and dimensioned to receive a third anode 172.

Fig. 6 displays how a prosthetic device 190 can have a first metal component 192 defining a first opening 196 configured and dimensioned to receive a first anode 194. A second metal component 198 defines at least one opening 202 configured dimensioned to receive a second anode 200. Unlike in Fig. 5, where the anodes are generally along the same surface or direction, the first opening 196 is oriented in a first direction D1 to receive the first anode 194, and the second opening 202 is oriented in a second direction D2 to receive the second anode 200.

From the embodiment of Fig. 6, the concept of the sacrificial anode in a prosthetic device can be applied along different surfaces to protect different components. The anodes and corresponding openings in the metal components can vary relative to one another, such as weight or surface area. As shown in Fig. 5, multiple anodes can be applied along different surfaces.

A kit can be provided for retrofitting a metal component in a prosthetic device. For example, while an opening in an adapter can be shaped for receiving a tool, as in a hex or star key, for adjusting the adapter, an anode can be correspondingly configured and dimensioned to fit within such opening once the adapted is suitably adjusted. Thus, the anode can be removed from the opening for further adjustment or replacement with a new anode to readjust the adapter.

A method may be provided to install or retrofit a metal component in a prosthetic device with an anode. The anode can be press-fitted into an opening formed by the metal component, or the method may involve forming an opening for accommodating a correspondingly sized anode. Such a method may involve the replacement or removal of the anode from the opening.

The embodiments and methods for determining the anodes and their locations provide improved corrosion resistance to metal components in prosthetic devices. The concepts of the anodes can be extended to other articles employing the same criteria or including different or additional criteria to improve corrosion resistance, such as in orthopedic devices.

It is to be understood that not necessarily all objects or advantages may be achieved under any embodiment of the disclosure. Those skilled in the art will recognize that the anode may be embodied or carried out so it achieves or optimizes one advantage or group of advantages as taught herein without necessarily achieving other objects or advantages as may be taught or suggested herein.

The skilled artisan will recognize the interchangeability of various disclosed features. Besides the variations described, other known equivalents for each feature can be mixed and matched by one of ordinary skill in this art to construct a prosthetic device with anodes under principles of the present disclosure. The skilled artisan will understand that the features described may be adapted to other types of articles. Hence this disclosure and the embodiments and variations thereof are not limited to prosthetic devices.

## Claims

1. A prosthetic device (100), comprising:
a metal component (112) mounted on a prosthetic foot or prosthetic knee;
**characterized by** a sacrificial anode (114, 126) is secured within an opening (124) to the metal component (112).

2. The prosthetic device (100) of claim 1, wherein the metal component (112) is mounted on a prosthetic foot or prosthetic knee, or any other prosthetic component including a metal feature (110).

3. The prosthetic device (100) of claim 1, wherein the anode (114, 126) is substantially smaller than the metal component (112).

4. The prosthetic device (100) of claim 1, wherein the metal component (112) is an adapter (120) having a locking part (122), optionally wherein the anode (126) is secured to the adapter (120) in a tight-fitting relationship, optionally wherein the anode (126) is press-fitted into the opening (124) and retained to the metal component by said press-fitting.

5. The prosthetic device (100) of claim 4, wherein the adapter (120) defines an extension (130), the locking part (122) defines the opening (124) configured and dimensioned to receive the anode (126) correspondingly shaped to the opening (124).

6. The prosthetic device (100) of claim 5, wherein the opening (124) defines a depth corresponding to a height (h) of the anode (126) such that a first end (132) of the anode (126) is flush with a surface (128) of the locking part (122), such that a surface area (134) of the first end (132) of the anode (126) is exposed.

7. The prosthetic device (100) of claim 6, wherein the surface area (134) of the anode (126) is substantially smaller than a surface area of the surface (128) of the locking part (122), optionally wherein the surface area (134) of the anode is at least 50% less, or more preferably less than 25%, than the surface area of the surface (128) of the locking part (122).

8. The prosthetic device (100) of claim 6, wherein the anode (126) defines a diameter (d) at least at the first end (132) that corresponds to a diameter or width of the opening (124) whereat it opens along the surface (128).

9. The prosthetic device (100) of claim 4, further comprising at least one further anode, wherein at least two of said anodes (126) are secured to the adapter (120).

10. The prosthetic device (100) of any one of the preceding claims, wherein the anode comprises a zinc alloy, optionally wherein the zinc alloy is at least 99% pure zinc.

11. The prosthetic device (100) of any one of the preceding claims, wherein the metal component is formed from at least one of the following metals selected from the group consisting of aluminum, titanium or stainless steel.

12. The prosthetic device (100) of claim 1, wherein the anode (150) defines a non-circular or cylindrical shape (152) configured and dimensioned to tightly fit within the opening (158) formed by a metal component (156) of a prosthetic component (154);
wherein the shape (152) corresponds to a shape formed by the opening (158).

13. The prosthetic device (100) of claim 12, wherein the shape (152) has an undulating profile.

14. The prosthetic device (100) of claim 12, wherein the anode (150) locks within the enclosure (158) defined by the metal component (156) by tightly fitting with the opening (158).

15. The prosthetic device (170) of claim 1, wherein the metal component is a first metal component (180) that defines first and second openings (178) configured and dimensioned to receive first and second anodes (176), or wherein the prosthetic device (190) comprises the metal component being a first metal component (192) defining a first opening (196) configured and dimensioned to receive a first anode (194), and further comprises a second metal component (198) defining at least one opening (202) configured and dimensioned to receive a second anode (200).

## Patentansprüche

1. Eine prothetische Vorrichtung (100), die Folgendes umfasst:
- eine Metallkomponente (112), die an einer Fußprothese oder einer Knieprothese angebracht ist;
- **gekennzeichnet durch** eine Opferanode (114, 126), die in einer Öffnung (124) an der Metallkomponente (112) befestigt ist.

2. Die prothetische Vorrichtung (100) nach Anspruch 1, wobei die Metallkomponente (112) auf einer Prothesenkomponente angebracht ist, die ein Metallelement (110) beinhaltet.

3. Die prothetische Vorrichtung (100) nach Anspruch 1, wobei die Anode (114, 126) wesentlich kleiner ist als die Metallkomponente (112).

4. Die prothetische Vorrichtung (100) nach Anspruch 1, wobei die Metallkomponente (112) ein Adapter (120) mit einem Verriegelungsteil (122) ist, wobei die Anode (126) gegebenenfalls eng an dem Adapter (120) befestigt ist und wobei die Anode (126) gegebenenfalls in die Öffnung (124) eingepresst und durch diese Einpressung an der Metallkomponente gehalten wird.

5. Die prothetische Vorrichtung (100) nach Anspruch 4, wobei der Adapter (120) eine Verlängerung (130) definiert und das Verriegelungsteil (122) die Öffnung (124) definiert, die so konfiguriert und dimensioniert ist, dass sie die entsprechend der Öffnung (124) geformte Anode (126) aufnimmt.

6. Die prothetische Vorrichtung (100) nach Anspruch 5, wobei die Öffnung (124) eine Tiefe definiert, die einer Höhe (h) der Anode (126) entspricht, sodass ein erstes Ende (132) der Anode (126) bündig mit einer Oberfläche (128) des Verriegelungsteils (122) abschließt und somit ein Oberflächenbereich (134) des ersten Endes (132) der Anode (126) freiliegt.

7. Die prothetische Vorrichtung (100) nach Anspruch 6, wobei der Oberflächenbereich (134) der Anode (126) wesentlich kleiner ist als ein Oberflächenbereich der Oberfläche (128) des Verriegelungsteils (122), wobei der Oberflächenbereich (134) der Anode gegebenenfalls mindestens 50 % kleiner oder bevorzugter kleiner als 25 %, als der Oberflächenbereich der Oberfläche (128) des Verriegelungsteils (122) ist.

8. Die prothetische Vorrichtung (100) nach Anspruch 6, wobei die Anode (126) mindestens an ihrem ersten Ende (132) einen Durchmesser (d) definiert, der einem Durchmesser oder einer Breite der Öffnung (124) entspricht, an der sie sich entlang der Oberfläche (128) öffnet.

9. Die prothetische Vorrichtung (100) nach Anspruch 4, weiter umfassend mindestens eine weitere Anode, wobei mindestens zwei dieser Anoden (126) an dem Adapter (120) befestigt sind.

10. Die prothetische Vorrichtung (100) nach einem der vorstehenden Ansprüche, wobei die Anode eine Zinklegierung umfasst, wobei die Zinklegierung gegebenenfalls zu mindestens 99 % reines Zink ist.

11. Die prothetische Vorrichtung (100) nach einem der vorstehenden Ansprüche, wobei die Metallkomponente aus mindestens einem der folgenden Metalle, ausgewählt aus der Gruppe bestehend aus Aluminium, Titan oder Edelstahl, gebildet ist.

12. Die prothetische Vorrichtung (100) nach Anspruch 1, wobei die Anode (150) eine nicht kreisförmige oder zylindrische Form (152) definiert, die so konfiguriert und dimensioniert ist, dass sie eng in der Öffnung (158) sitzt, die von einer Metallkomponente (156) einer Prothesenkomponente (154) gebildet wird;
- wobei die Form (152) einer durch die Öffnung (158) gebildeten Form entspricht.

13. Die prothetische Vorrichtung (100) nach Anspruch 12, wobei die Form (152) ein wellenförmiges Profil aufweist.

14. Die prothetische Vorrichtung (100) nach Anspruch 12, wobei die Anode (150) innerhalb des durch die Metallkomponente (156) gebildeten Gehäuses (158) durch engen Sitz in der Öffnung (158) verriegelt wird.

15. Die prothetische Vorrichtung (170) nach Anspruch 1, wobei die Metallkomponente eine erste Metallkomponente (180) ist, die eine erste und zweite Öffnung (178) definiert, die zum Aufnehmen einer ersten und einer zweiten Anode (176) konfiguriert und dimensioniert sind, oder wobei die prothetische Vorrichtung (190) die Metallkomponente umfasst, die eine erste Metallkomponente (192) ist, die eine erste Öffnung (196) definiert, die zum Aufnehmen einer ersten Anode (194) konfiguriert und dimensioniert ist, weiter umfassend eine zweite Metallkomponente (198), die mindestens eine Öffnung (202) definiert, die zum Aufnehmen einer zweiten Anode (200) konfiguriert und dimensioniert ist.

## Revendications

1. Dispositif prothétique (100), comprenant :
un composant métallique (112) monté sur un pied prothétique ou un genou prothétique ;
**caractérisé par**
une anode sacrificielle (114, 126) fixée à l'intérieur d'une ouverture (124) du composant métallique (112).

2. Dispositif prothétique (100) selon la revendication 1, dans lequel le composant métallique (112) est monté sur un composant prothétique comprenant un élément métallique (110).

3. Dispositif prothétique (100) selon la revendication 1, dans lequel l'anode (114, 126) est sensiblement plus petite que le composant métallique (112).

4. Dispositif prothétique (100) selon la revendication 1, dans lequel le composant métallique (112) est un adaptateur (120) comportant une partie de verrouillage (122), éventuellement dans lequel l'anode (126) est fixée à l'adaptateur (120) selon une relation d'ajustement serré, éventuellement dans lequel l'anode (126) est emmanchée à force dans l'ouverture (124) et retenue au composant métallique par ledit emmanchement à force.

5. Dispositif prothétique (100) selon la revendication 4, dans lequel l'adaptateur (120) définit une extension (130), la partie de verrouillage (122) définit l'ouverture (124) configurée et dimensionnée pour recevoir l'anode (126) de forme correspondant à l'ouverture (124).

6. Dispositif prothétique (100) selon la revendication 5, dans lequel l'ouverture (124) définit une profondeur correspondant à une hauteur (h) de l'anode (126) de sorte qu'une première extrémité (132) de l'anode (126) affleure une surface (128) de la partie de verrouillage (122), de sorte qu'une aire de surface (134) de la première extrémité (132) de l'anode (126) est exposée.

7. Dispositif prothétique (100) selon la revendication 6, dans lequel l'aire de surface (134) de l'anode (126) est sensiblement plus petite qu'une aire de surface de la surface (128) de la partie de verrouillage (122), éventuellement dans lequel l'aire de surface (134) de l'anode est inférieure d'au moins 50 %, ou plus préférentiellement inférieure de moins de 25 %, à l'aire de surface de la surface (128) de la partie de verrouillage (122).

8. Dispositif prothétique (100) selon la revendication 6, dans lequel l'anode (126) définit un diamètre (d) au moins au niveau de la première extrémité (132) qui correspond à un diamètre ou à une largeur de l'ouverture (124) à l'endroit où elle débouche le long de la surface (128).

9. Dispositif prothétique (100) selon la revendication 4, comprenant en outre au moins une autre anode, dans lequel au moins deux desdites anodes (126) sont fixées à l'adaptateur (120).

10. Dispositif prothétique (100) selon l'une quelconque des revendications précédentes, dans lequel l'anode comprend un alliage de zinc, éventuellement dans lequel l'alliage de zinc est constitué d'au moins 99 % de zinc pur.

11. Dispositif prothétique (100) selon l'une quelconque des revendications précédentes, dans lequel le composant métallique est formé d'au moins l'un des métaux suivants choisis dans le groupe constitué de l'aluminium, du titane ou de l'acier inoxydable.

12. Dispositif prothétique (100) selon la revendication 1, dans lequel l'anode (150) définit une forme non circulaire ou cylindrique (152) configurée et dimensionnée pour s'ajuster de manière serrée à l'intérieur de l'ouverture (158) formée par un composant métallique (156) d'un composant prothétique (154) ;
dans lequel la forme (152) correspond à une forme formée par l'ouverture (158).

13. Dispositif prothétique (100) selon la revendication 12, dans lequel la forme (152) présente un profil ondulé.

14. Dispositif prothétique (100) selon la revendication 12, dans lequel l'anode (150) se verrouille à l'intérieur de l'enceinte (158) définie par le composant métallique (156) en s'ajustant de manière serrée à l'ouverture (158).

15. Dispositif prothétique (170) selon la revendication 1, dans lequel le composant métallique est un premier composant métallique (180) qui définit des première et seconde ouvertures (178) configurées et dimensionnées pour recevoir des première et seconde anodes (176), ou dans lequel le dispositif prothétique (190) comprend le composant métallique étant un premier composant métallique (192) définissant une première ouverture (196) configurée et dimensionnée pour recevoir une première anode (194), et comprend en outre un second composant métallique (198) définissant au moins une ouverture (202) configurée et dimensionnée pour recevoir une seconde anode (200).
